(19) **Europäisches Patentamt**

**European Patent Office**

**Office européen des brevets**

(11) **EP 0 936 902 B1**

(12) **EUROPEAN PATENT SPECIFICATION**

(45) Date of publication and mention
of the grant of the patent:
**18.06.2003 Bulletin 2003/25**

(51) Int Cl.⁷: **A61K 9/16**, A61K 9/48,
A61K 9/50, A61K 9/72,
A61K 48/00

(21) Application number: **97915601.5**

(22) Date of filing: **03.04.1997**

(86) International application number:
**PCT/GB97/00953**

(87) International publication number:
**WO 97/036578 (09.10.1997 Gazette 1997/43)**

(54) **SPRAY-DRIED MICROPARTICLES AS THERAPEUTIC VEHICLES FOR USE IN GENE THERAPY**

SPRÜHGETROCKNETE MIKROPARTIKEL ALS THERAPEUTISCHE TRÄGER

MICROPARTICULES SECHEES PAR PULVERISATION, SERVANT DE VEHICULES THERAPEUTIQUES EN THERAPIE GENETIQUE

(84) Designated Contracting States:
**AT BE CH DE DK ES FI FR GB GR IE IT LI LU MC NL PT SE**

(30) Priority: **03.04.1996 GB 9607035**
**07.06.1996 WOPCT/GB96/01379**

(43) Date of publication of application:
**25.08.1999 Bulletin 1999/34**

(73) Proprietor: **Elan Drug Delivery Limited**
**Nottingham, NG11 6JS (GB)**

(72) Inventors:
• **SUTTON, Andrew Derek**
**Grantham NG31 8FY (GB)**
• **OGDEN, Jill Elizabeth**
**Ruddington, Nottingham NG11 6JS (GB)**
• **JOHNSON, Richard Alan**
**Ruddington, Nottingham NG11 6JS (GB)**

(74) Representative: **Perry, Robert Edward**
**GILL JENNINGS & EVERY**
**Broadgate House**
**7 Eldon Street**
**London EC2M 7LH (GB)**

(56) References cited:
EP-A- 0 606 486          EP-A- 0 611 567
WO-A-92/18164           WO-A-94/23699
WO-A-95/31479           WO-A-96/09814
WO-A-96/27393           WO-A-97/03702

• Eur. J. Pharm. Biopharm., Volume 40, No. 4,
1994, Ubaldo Conte et al, "Spray Dried Albumin
Microspheres Containing Nicardipine"

**Description**

Field of the Invention

This invention relates to spray-dried microparticles and their use as therapeutic vehicles. In particular, the invention relates to means for delivery of agents for gene therapy.

Background of the Invention

Current methods of aerosolising drugs for inhalation include nebulisation, metered dose inhalers and dry powder inhaler systems. Nebulisation of aqueous solutions and suspensions requires large volumes of drugs and involves the use of bulky and non-portable devices.

The most common method of administration of therapeutic agents to the lung is by the use of volatile propellant-based devices, commonly termed metered dose inhalers. A solution of propellant, commonly CFC 11, 12 or 114, is used, containing either dissolved drug or a suspension of the drug in a pressurised canister. Dosing is achieved by depressing an actuator which releases a propellant aerosol of drug suspension or solution which is carried on the airways. During its passage to the lung, the propellant evaporates to yield microscopic precipitates from solution or free particles from suspension. The dosing is fairly reproducible and cheap, but there is growing environmental pressure to reduce the use of CFCs. CFCs will shortly be replaced by non-ozone-depleting propellants, so-called hydrofluoroalkanes. Furthermore, the use of CFC solvents remains largely incompatible with many drugs, because of their susceptibility to denaturation and low stability.

Concurrently, there is a move towards using dry powder inhalers (DPIs) containing drugs, usually admixed with a carrier, such as mannitol, lactose or glucose, which facilitates the aerosolisation and dispersion of the drug particles. The energy for disaggregation is often supplied by the breath or inspiration of air through the device by the patient.

Drugs are currently micronised, to reduce particle size. This approach is not applicable for biotechnology products. In general, biotechnology products are available in low quantity and, furthermore, are susceptible to the methods currently employed to dry and micronise drugs, prior to mixing with carrier. Further, it is particularly difficult to provide blends of drug and carrier which are sufficiently free-flowing that they flow and dose reproducibly in modern multiple dose inhalers such as the Turbohaler (Astra) and Diskhaler (Glaxo).

WO-A-9116882 describes the use of spray-drying to produce lipid/drug powders of conventional asthma drugs which purportedly showed a sustained release effect due to the presence of the lipid. The conditions given for spray-drying produce dry powders which are bimodally distributed, with peaks at 1 μm and 7-10 μm.

WO-A-8806441 describes the use of spray-drying in the production of stable liposome formulations. A preserving additive such as a sugar is spray-dried with the lipid material. The liposomes retain their size distribution and integrity, on reconstitution. The stabilised liposomes may contain a therapeutic agent, apparently for use after storage of the liposomes and the removal of the preserving additive.

1-5 μm is the optimal particle size for penetration to the alveolar regions. Adsorption in the alveolar region offers the largest surface area for access to the bloodstream, and therefore it is desirable that a dry powder formulation should contain a large preponderance of particles with these dimensions. It has proved difficult to produce particles in this range reproducibly by micronisation techniques. Whilst there have been preliminary reports on the use of spray-drying to produce respirable powders, the first description of the precise process conditions leading to particles for therapeutic use, having satisfactory narrow interquartile ranges, in the 1-5 μm range, is in WO-A-9609814 (Andaris).

While microparticles in the 1-5 μm range are of optimal size for alveolar penetration when aerosolised as discrete particles, they do not always possess the necessary bulk powder flow properties which are required for DPI device filling and dose dispensing. Particles of this size are highly cohesive and will generally require coarser-sized particles to impart the bulk flow properties required; larger particles flow better. For drugs that have low potency, dispensing and blending with a conventional carrier, e.g. lactose, present few problems in terms of reproducibility and accuracy. For highly potent drugs, however, accurate blending and dispensing becomes a real problem. When the required drug dose may be as small as 1 μg, contained in an emitted mass of 12-25 mg, the problem of accurate dosing becomes a real impediment to the development of such highly potent drugs.

Summary of the Invention

Microparticles according to the present invention, of which at least 90% can have a size of 1-10 μm, are intended. for use in gene therapy, and comprise a cationic material and a substantially uniform mixture of DNA, as an agent for gene therapy, and an excipient.

Microparticles of this invention may be formulated with a carrier ("carrier" is used herein for distinction from the "excipient" in the microparticles). The formulation may be administered, e.g. from a dry powder inhaler. On administration, the carrier disperses in the airstream; because of its large size, it is deposited in the mouth and throat. The smaller microparticles go to the peripheral airways; on reaching the lung, the excipient dissolves and its diluting effect is lost. The dispersed therapeutic agent is reproducibly dosed to the desired locus of administration.

Description of the Invention

Microparticles of this invention may be obtained by spray-drying under conditions described in WO-A-9609814. Formulations containing them overcome some of the fundamental problems associated with dry powders for DPIs, specifically, poor powder mixing and limited accuracy of blending and dosing for highly potent drugs.

Particles of the same size are also suitable for intravenous administration, e.g. for targeted delivery to the liver, and for gene therapy. Soluble microparticles prepared by spray-drying may be cross-linked, by known technology, for this purpose. By comparison with, say, iv administration of adenovirus, targeted administration is achieved by means of the invention.

More generally, a process for preparing microcapsules of the invention comprises atomising a solution (or dispersion) of excipient and a therapeutic agent that is itself, or is used together with, a wall-forming material. In particular, it has been found that, under the conditions stated herein, and more generally described in WO-A-9218164, remarkably smooth spherical microparticles of various materials may be produced. The particle size and size distribution of the product can be reproducibly controlled within a tight range. For example, by Coulter analysis, 98% of the particles can be smaller than 6 µm on a number basis, within an interquartile range of 2 µm, and with less than 0.5 µm mean size variation between batches. Furthermore, when tested in a dry powder inhaler, reproducible dosing was achieved, and subsequent aerosolisation under normal but low flow conditions (30 1/min) resulted in excellent separation of microparticles from carrier.

The therapeutic agent that is used in this invention is intended for gene therapy. It may be a naked or encapsulated gene. Alternatively, it may be a virus particle that is stabilised by the presence of the excipient. Agents suitable for use in gene therapy are receptor-targeted complexes, adenovirus, adeno-associated virus, retrovirus, parvovirus, vaccinia virus, herpes virus and sindbis virus. Cationic lipid-DNA complexes may be used.

Antisense oligonucleotides may also be administered by means of this invention. For example, the oligonucleotide may be chosen to degenerate the template activity of template RNAs. It is demonstrated by Nyce and Metzger, Nature $\underline{385}$:721-5 (1997), that aerosolised phosphorothioate antisense oligonucleotide targeting the adenosine $A_1$ receptor desensitised rabbits to subsequent challenge with adenosine or dust-mite allergen, and is therefore of potential utility in the treatment of asthma.

DNA being anionic, microparticles of the invention may comprise a cationic material. In particular, cationic lipids capable of forming positively-charged liposomes have the ability to bind DNA and can be used for gene delivery to mammalian cells both *in vitro* and *in vivo.* By way of example, DNA may be encapsulated by binding to lipid domains in a lactose or other carrier. Ideally, the lipid domains should be <1 µm and the microcapsules of the order of 2-5 µm, for deposition in the peripheral airways. The principle has been tested using control lipids and control DNA and the methodology transferred to produce an active plasmid DNA/lipid/lactose microcapsule using the lipids DC-Chol and DOPE and marker plasmid.

Other suitable cationic materials are chitosan and lysine. Lysine has a very polar side-chain which is positively charged at neutral pH and highly hydrophilic. These properties make it suitable for co-spray-drying with HSA (human serum albumin), to form microcapsules. Successful DNA binding has been achieved on microparticles containing 5% w/w of 25% lysine:75% HSA.

Chitosan is a natural polysaccharide suitable for biomedical applications. Lysozyme will biodegrade chitosan *in vivo.* By cross-linking the polymer matrix using heat or chemical methods, e.g. glycolaldehyde, chitosan becomes less susceptible to lysosomal degradation and thus suitable for prolonged delivery of drugs. The cationic property of the chitosan has proved to bind DNA.

The excipient will usually be chosen on account of its ability to dilute/stabilise the therapeutic agent, and because of its wall-forming properties. It should be capable of formulation for spray-drying. Suitable excipients include carbohydrates, e.g. sugars such as glucose, lactose and mannitol. α-Lactose and mannitol may have the particularly desirable property of resisting moisture uptake. See also EP-A-0372777.

The amount of excipient in the mixture of the microparticles will usually be chosen having regard to the desired degree of, say, dilution or stabilisation. It may be at least 50% by weight of the mixture, and often at least 70% or 80%.

Another inert wall-forming, material may also be used, if desired, to form the microparticles. Suitable such materials are water-soluble.

The wall-forming material and process conditions should be so chosen that the product is sufficiently non-toxic and non-immunogenic in the conditions of use, which will clearly depend on the dose administered and duration of treatment. The wall-forming material may be a starch derivative, a synthetic polymer such as tert-butyloxycarbonylmethyl polyglutamate (US-A-4888398) or a polysaccharide such as polydextrose. Generally, the wall-forming material can be selected from most hydrophilic, biodegradable physiologically compatible polymers, as described in more detail in WO-A-9218164.

Especially for pulmonary delivery, the microparticles are preferably soluble, and preferably comprise a carbohydrate excipient. A sugar may facilitate dissolution of the microparticles on the lung surface.

For parenteral administration in particular, the microparticles are preferably stabilised, e.g. cross-linked by chemical means or heat. In this case, a wall-forming material used for gene therapy may be proteinaceous. For example, it may be collagen, gelatin or (serum) albumin, in each case preferably of human origin (i.e. derived from humans or corresponding in structure to the human protein). Most preferably, it is HSA derived from blood donations or, ideally, derived recombinantly from the fermentation of microorganisms (including cell lines) which have been transformed or transfected to express recombinant serum albumin. Further detail is given in WO-A-9218164.

For use in a DPI, microparticles of the invention may be mixed with a conventional carrier such as mannitol, lactose or glucose (which may be the same as the excipient). The carrier will have a particle size, and be in an amount, chosen in accordance with approved usage of the device. By way of example, 3 µm microparticles of the invention may be mixed with lactose particles 80 µm in size.

There are various considerations, in determining an appropriate percentage of the agent to total solids in the spray-drying medium. These criteria include the dose of the agent from one "shot" of the device; the dispensed weight of the formulation, i.e. the amount of powder in one "shot"; and the microparticle:carrier ratio, i.e. the proportion of microcapsules to carrier.

The spray-drying process can be controlled in order to obtain microspheres with desired characteristics. Thus, the pressure at which the seed solution is supplied to the spray nozzle may be varied, for example from $1.0\text{-}10.0 \times 10^5$ Pa, preferably $2\text{-}8 \times 10^5$ Pa, and most preferably about $7.5 \times 10^5$ Pa. Other parameters may be varied as disclosed below.

More than 30%, preferably more than 40%, 50%, or 60%, of the microspheres have a diameter within a 2 µm range and at least 90%, preferably at least 95% or 99%, have a diameter within the range 1.0-8.0 µm. The interquartile range may be 2 µm, with a median diameter of 3.5, 4.0, 4.5, 5.0, 5.5, 6.0 or 6.5 µm.

Thus, at least 30%, 40%, 50% or 60% of the microspheres may have diameters within the range 1.5-3.5 µm, 2.0-4.0 µm, 3.0-5.0 µm, 4.0-6.0 µm, 5.0-7.0 µm or 6.0-8.0 µm. Preferably, a said percentage of the microspheres have diameters within a 1.0 µm range, such as 1.5-2.5 µm, 2.0-3.0 µm, 3.0-4.0 µm, 4.0-5.0 µm, 5.0-6.0 µm, 6.0-7.0 µm or 7.0-8.0 µm.

A product of the invention may be in the form of hollow microspheres, in which at least 90%, preferably at least 95% or 99%, of the microspheres have a diameter in the range 1.0-8.0 µm.

For spray-drying, a solution, suspension, emulsion or dispersion preferably contains 0.1 to 50% w/v, more preferably about 5.0-25.0%, of the materials of microcapsules. About 20% is optimal.

The solution or dispersion (preferably solution), referred to herein as the "seed solution", is atomised and spray-dried by any suitable technique which results in discrete microspheres or microcapsules of 1 to 10 µm diameter. These figures refer to at least 90% of the population of microcapsules, the diameter being measured with a Coulter Multisizer II. The term "microcapsules" means hollow particles enclosing a space, which space is filled with a gas or vapour but not with any solid materials.

The atomising comprises forming an aerosol of the preparation by, for example, forcing the preparation through at least one orifice under pressure into, or by using a centrifugal atomiser in a chamber of warm air or other inert gas. The chamber should be big enough for the largest ejected drops not to strike the walls before drying. The gas or vapour in the chamber is clean (i.e. preferably sterile and pyrogen-free) and non-toxic when administered into the bloodstream in the amounts concomitant with administration of the microcapsules in use. The rate of evaporation of the liquid from the preparation should be sufficiently high to form hollow microcapsules but not so high as to burst the microcapsules. The rate of evaporation may be controlled by varying the gas flow rate, concentration of drug and excipient in the seed solution, nature of liquid carrier, feed rate of the solution and, more importantly, the temperature of the gas encountered by the aerosol. With an albumin concentration of 15-25% in water, an inlet gas temperature of at least about 100°C, preferably at least 110°C, is generally sufficient to ensure hollowness and the temperature may be as high as 250°C without the capsules bursting. About 180-240°C, preferably about 210-230°C and most preferably about 220°C, is optimal, at least for albumin. Since the temperature of the gas encountered by the aerosol will depend also on the rate at which the aerosol is delivered and on the liquid content of the protein preparation, the outlet temperature may be monitored to ensure an adequate temperature in the chamber. An outlet temperature of 40-150°C has been found to be suitable. Controlling the flow rate has been found to be useful in controlling the other parameters such as the number of intact hollow particles.

Due to the heat-sensitive nature of DNA, it would be advantageous to spray-dry using a low outlet temperature. An increase in size has been observed, on reducing the temperature. This size increase may be due to the microcapsules having a higher moisture content which in turn results in particle agglomeration. To avoid this agglomeration, an outlet temperature of 70°C is favoured.

More particularly, microparticles of the invention preferably have a maximum interquartile range of 3 µm, more preferably 2 µm, and most preferably 1.5 µm, with respect to their volume median particle size. The volume median particle diameter is determined by Coulter counter. This is achieved by spray-drying in which there is a combination of low feed stock flow rate with high levels of atomisation and drying air. The effect is to produce microcapsules of very defined size and tight size distribution.

Several workers have designed equations to define the mean droplet size of pneumatic nozzles; a simple version of the various parameters which affect mean droplet size is as follows:

$$D = A/(V^2.d)^a + B. (M_{air}/M_{liq})^{-b}$$

Where

| | |
|---|---|
| D = | Mean droplet size |
| A = | Constant related to nozzle design |
| B = | Constant related to liquid viscosity |
| V = | Relative air velocity between liquid and nozzle |
| d = | Air density |
| $M_{air}$ and $M_{liq}$ = | Mass of air and liquid flow |
| a and b = | Constants related to nozzle design |

Clearly, for any given nozzle design, the droplet size is most affected by the relative velocity at the nozzle and concurrently the mass ratio of air to liquid. For most common drying use, the air to liquid ratio is in the range of 0.1-10 and at these ratios it appears that the average droplet size is 15-20 µm. For the production of microparticles in the size range described herein, the air to liquid ratio is preferably from 20-1000:1. The effect is to produce particles at the high ratios which are exceedingly small by comparative standards, with very narrow size distributions. For microparticles, produced at the lower ratios of air to liquid, slightly larger particles are produced, but they still nevertheless have tight size distributions which are superior to microparticles produced by emulsion techniques.

The invention allows for the nature of the dry microcapsules to be manipulated, in order to optimise the flow or vehicle properties, by changing and reducing the forces of cohesion and adhesion within the microcapsule preparation. For example, if so required, the microcapsules may be made predominantly positive or negative by the use of highly-charged monomeric or polymeric materials, e.g. lysine or poly-lysine and glutamate or poly-glutamate in systems without HSA or heterogeneous systems including HSA and active principles.

In the Examples, dry powder formulations of gene vectors suitable for pulmonary delivery using a dry powder inhaler device were prepared by spray-drying. The resulting microparticles comprised the gene vector (cationic lipids: DNA complexes or adenovirus) dispersed within the sugar excipient shell. The sugar excipient component of the microparticles enables their dissolution on the lung surface, thereby releasing the gene vector at the optimal site within the lung.

The gene vectors carried reporter genes encoding luciferase of β-galactosidase to investigate DNA activity through the spray drying process. Gene transfection assays were performed on the dry powder formulations of both types of vectors, using in vitro cell culture methods.

Example 1

Plasmid pCMVLuc (CMV promoter:luciferase gene) was reconstituted with the lipids DC-Chol/DOPE at a charge ratio of 5:1. DC-Chol is 3β-[N-(N',N'-dimethylaminoethane)-carbamoyl]cholesterol; DOPE is dioleoylphosphatidylethanolamine.

A feedstock for spray-drying was prepared containing 1 mg plasmid DNA, 8 mg DC-Chol, 8 mg DOPE and 700 mg lactose as excipient, in 7 ml water. The feed solution was spray-dried using a laboratory bench top spray-dryer, under the following conditions:

| | |
|---|---|
| Inlet temperature | 130°C |
| Outlet temperature | 70.1°C |
| Atomisation pressure | 3.0 bar |
| Feed rate | 0.75 g/min |

200 mg of spray-dried product was obtained, equivalent to a recovery of 35%. Analysis of the finished product using a Coulter LS230 Laser Sizer demonstrated that the lactose:cationic lipid:DNA microparticles had a median particle diameter (volume) of 3.8 $\mu$m. Typically, the cationic lipid:DNA domains were less than 200 nm in size.

Restriction analysis of plasmid DNA extracted from the microparticles using standard procedures demonstrated that the restriction pattern of the spray-dried DNA was identical to that of the control plasmid.

Two such samples (1A and 1B) were prepared. Microcapsules were also prepared comprising lactose only (1C) and lactose:cationic lipid (1D). A549 cells (derived from a human lung tumour) were grown in 60 mm diameter cell culture dishes. Cells were seeded at $5 \times 10^5$ the day before transfection. To mimic delivery to the lung, i.e. the use of intra-tracheal powder aerosolisation, the microparticles/microcapsules were sprayed as a dry powder onto the surface of the cells using a syringe device after aspiration of the tissue culture medium. It was estimated that less than 10 $\mu$g pCMVLuc DNA was added to each dish. After 30 min, 2 ml fresh cell culture medium was added. At 48 hr post-transfection, cells were lysed in 500 $\mu$l lysis buffer (Promega), and the luciferase activity was measured in 20 $\mu$l aliquots of cell lysate.

The positive controls were DC-Chol/DOPE (40 $\mu$g):pCMVLuc (10 $\mu$g) in Experiment 1 and Spermidine-Chol/DOPE (40 $\mu$g):pCMVLuc (10 $\mu$g) in Experiment 2. Gene transfection data, expressed in terms of luciferase activity (RLU/min), are given in Table 1. Two values are given, where luciferase activity was measured in two independent dishes.

Table 1

| SAMPLE | EXPERIMENT 1 (RLU/min) | EXPERIMENT 2 (RLU/min) |
|---|---:|---:|
| Control Cells | 826<br>776 | 792 |
| Lactose | 830<br>736 | |
| pCMVLuc | 760<br>810 | |
| +ve Control | 8636412<br>9896528 | 4192473<br>3490039 |
| 1A | 125984<br>180776 | 32084<br>241805 |
| 1B | 2051592 | 7505<br>31604 |
| 1C | 1392<br>1394 | |
| 1D | 878 | 771 |

Example 2

A plasmid carrying the $\beta$-galactosidase gene (CMV promoter) as reporter was reconstituted with the lipid DOTAP, i.e. N-[1-(2,3-dioleoyloxy)propyl]-N,N,N-trimethylammonium methylsulphate, and spray-dried with mannitol as excipient. The feedstock contained 2 mg plasmid DNA, 10 mg DOPE and 388 mg mannitol, in 4 ml diluent. The feed solution was spray-dried using a bench top spray dryer under the following conditions:

| | |
|---|---|
| Inlet temperature | 130°C |
| Outlet temperature | 78°C |
| Atomisation pressure | 3.0 bar |
| Feed rate | 0.72 g/min |

175 mg of spray-dried product was obtained, equivalent to a recovery of 43.8%. Analyses of the spray-dried product on a Coulter LS230 Laser Sizer demonstrated that the microparticles had a median diameter (volume) of 3-6 $\mu$m.

Standard procedures were used to assess gene transfection efficiency. B16 mouse melanoma cells were seeded at $10^5$ cells per well and incubated for 24 hr. Samples of cationic lipid:plasmid DNA:mannitol microparticles containing 2 µg plasmid DNA were dissolved in water and used to transfect the confluent cells. The controls were 2 µg plasmid DNA only and DOTAP/DNA at 2 µg DNA. The cells were incubated in the transfection solution for 4 hr and then grown for a further 48 hr before harvesting. Protein extracts were prepared and these were analysed for β-galactosidase activity. Transfection efficiencies in terms of β-galactosidase activity are given in Table 2.

Table 2

| Sample | β-galactosidase activity (mU/mg protein) | Mean | Standard Deviation |
|---|---|---|---|
| Plasmid DNA only | 3.41<br>2.32<br>2.08 | 2.60 | 0.71 |
| DOTAP/DNA | 10000.18<br>9803.90<br>10142.92 | 9982.33 | 170.21 |
| DOTAP/DNA/ mannitol microparticles | 6530.99<br>5043.20<br>5063.85 | 5546.01 | 853.08 |

Example 3

A feedstock was prepared containing an adenoviral vector (luciferase gene as reporter molecule) at a titre of $5.3 \times 10^{10}$ IU/ml ($7.8 \times 10^{10}$PFU/ml) in 10 mM Tris, 1 mM $MgCl_2$, 150 mM, Tween 80 (54 mg/ml) and 1M sucrose, at pH 8.5. The feedstock (50 ml), at a concentration of 0.352 g/ml, was spray-dried using a laboratory bench top spray-dryer under the following conditions:

| | |
|---|---|
| Inlet temperature | 56°C |
| Outlet temperature | 39.9°C |
| Atomisation pressure | 3.0 bar |
| Feed rate | 0.75 g/min |

The recovery of product after spray drying is summarised in Table 3.

Table 3

| | Before Spray-Drying | Post-Spray-Drying | Recovery |
|---|---|---|---|
| Mass (g) | 17.62 | 6.46 | 36.6% |
| Titre (IU/ml) | $5.3 \times 10^{10}$ | $2.6 \times 10^{10}$ | 49.0% |

The dry powder microparticle formulation of adenovirus was investigated for gene transfection activity in an *in vitro* cell culture assay using A549 cells. The microparticles were dissolved in water, and samples equivalent to 0.5 mg, 2 mg, 5 mg and 20 mg dry powder were used to transfect the cells. Adenovirus at a MOI (multiplicity of infection) of 100 PFU/cell was used as control. All samples were assayed in duplicate. The efficiency of gene transfection measured in luciferase activity is given in Table 4.

Table 4

| Sample | Luciferase Activity (RLU/min) | |
|---|---|---|
| Adenovirus control | 17473732 | 17949356 |
| Adenovirus control | 26138380 | 27845016 |
| +20 mq dry powder | 42104272 | 42878048 |
| +2 mg dry powder | 13697072 | 13401940 |

Table 4   (continued)

| Sample | Luciferase Activity (RLU/min) | |
|---|---|---|
| +0.5 mg dry powder | 5214460 | 5816246 |
| +5 mg dry powder | 24963076 | 24989568 |
| Control | 760 | 574 |
| Control | 203 | 183 |

These data demonstrate that the adenovirus retains significant gene transfection activity.

## Claims

1. Microparticles, which are smooth and spherical, at least 90% of which have a particle size of 1 to 10 µm, and which comprise a cationic material and a substantially uniform mixture of DNA, as an agent for gene therapy, and an excipient.

2. Microparticles according to claim 1, wherein the excipient constitutes a major proportion of the mixture.

3. Microparticles according to claim 1 or claim 2, obtainable by spray-drying a solution of the cationic material, the agent and the exipient.

4. Microparticles according to any preceding claim, wherein said particle size is 1 to 5 µm.

5. Microparticles according to any preceding claim, which have a maximum interquartile range of 3 µm.

6. Microparticles according to claim 5, which have a maximum interquartile range of 2 µm.

7. Microparticles according to any preceding claim, obtainable by an aseptic process.

8. Microparticles according to any preceding claim, wherein the excipient comprises a carbohydrate.

9. Microparticles according to any preceding claim, wherein said agent is a naked or encapsulated gene, or a virus particle.

10. Microparticles according to any preceding claim, wherein the cationic material comprises lipid domains.

11. A free-flowing particulate composition comprising microparticles according to any preceding claim and relatively large particles of a carrier material which may be the same as or different from the excipient.

12. An inhaler device adapted to deliver a therapeutic agent via the pulmonary airways, which comprises a composition according to claim 11.

13. Use of DNA capable of gene therapy, for the manufacture of a medicament for treatment of a complaint on which the said agent acts on administration *via* the pulmonary airways, **characterised in that** the medicament is in the form of microparticles according to any of claims 1 to 10 or a composition according to claim 11.

## Patentansprüche

1. Glatte und sphärische Mikropartikel, von denen mindestens 90 % eine Partikelgröße von 1 bis 10 µm aufweisen und welche ein kationisches Material und eine im wesentlichen gleichförmige Mischung von DNA, als Mittel zur Gentherapie, und einem Exzipienten umfassen.

2. Mikropartikel nach Anspruch 1, wobei der Exzipient einen Hauptteil der Mischung ausmacht.

3. Mikropartikel nach Anspruch 1 oder Anspruch 2, erhältlich durch Sprühtrocknung einer Lösung des kationischen Materials, des Mittels und des Exzipienten.

4. Mikropartikel nach irgendeinem der vorhergehenden Ansprüche, wobei die Partikelgröße 1 bis 5 µm beträgt.

5. Mikropartikel nach irgendeinem der vorhergehenden Ansprüche, welche einen maximalen Interquartilbereich von 3 µm aufweisen.

6. Mikropartikel nach Anspruch 5, welche einen maximalen Interquartilbereich von 2 µm aufweisen.

7. Mikropartikel nach irgendeinem der vorhergehenden Ansprüche, erhältlich durch ein steriles Verfahren.

8. Mikropartikel nach irgendeinem der vorhergehenden Ansprüche, wobei der Exzipient ein Kohlenhydrat umfaßt.

9. Mikropartikel nach irgendeinem der vorhergehenden Ansprüche, wobei das Mittel ein nacktes oder umhülltes Gen oder ein Viruspartikel ist.

10. Mikropartikel nach irgendeinem der vorhergehenden Ansprüche, wobei das kationische Material Lipid-Domänen umfaßt.

11. Freifließende Zusammensetzung in Teilchenform, umfassend Mikropartikel nach irgendeinem der vorhergehenden Ansprüche und relativ große Partikel eines Trägermaterials, welches das gleiche wie der Exzipient oder davon verschieden sein kann.

12. Inhalationsvorrichtung, ausgelegt zur Verabreichung eines therapeutischen Mittels über die Lungenwege, welches eine Zusammensetzung nach Anspruch 11 umfaßt.

13. Verwendung von DNA, die zur Gentherapie in der Lage ist, zur Herstellung eines Medikaments zur Behandlung eines Leidens, auf welches das Mittel nach Verabreichung über die Lungenwege einwirkt, **dadurch gekennzeichnet, daß** das Medikament in Form von Mikropartikeln nach irgendeinem der Ansprüche 1 bis 10 oder einer Zusammensetzung nach Anspruch 11 vorliegt.

**Revendications**

1. Microparticules, qui sont lisses et sphériques, dont au moins 90% ont une granulométrie de 1 à 10 µm, et qui comprennent une matière cationique et un mélange essentiellement uniforme d'ADN, comme agent pour une thérapie génique, et un excipient.

2. Microparticules selon la revendication 1, dans lesquelles l'excipient constitue une proportion majeure du mélange.

3. Microparticules selon la revendication 1 ou la revendication 2, pouvant être obtenues par un séchage par atomisation d'une solution de la matière cationique, de l'agent et de l'excipient.

4. Microparticules selon l'une quelconque des revendications précédentes, dans lesquelles ladite granulométrie est de 1 à 5 µm.

5. Microparticules selon l'une quelconque des revendications précédentes, qui ont un écart interquartile maximal de 3 µm.

6. Microparticules selon la revendication 5, qui ont un écart interquartile maximal de 2 µm.

7. Microparticules selon l'une quelconque des revendications précédentes, pouvant être obtenues par un procédé aseptique.

8. Microparticules selon l'une quelconque des revendications précédentes, dans lesquelles l'excipient comprend un hydrate de carbone.

**9.** Microparticules selon l'une quelconque des revendications précédentes, dans lesquelles ledit agent est un gène nu ou encapsulé, ou une particule de virus.

**10.** Microparticules selon l'une quelconque des revendications précédentes, dans lesquelles la matière cationique comprend des domaines lipidiques.

**11.** Composition particulaire à haute fluidité, comprenant des microparticules selon l'une quelconque des revendications précédentes, et des particules relativement grosses d'une matière de transport qui peut être la même, ou être différente de l'excipient.

**12.** Dispositif d'inhalation adapté pour dispenser un agent thérapeutique via les voies aériennes pulmonaires, qui comprend une composition selon la revendication 11.

**13.** Utilisation d'un ADN capable d'une thérapie génique, pour la fabrication d'un médicament pour le traitement d'un symptôme sur lequel ledit agent agit lors d'une administration via les voies aériennes pulmonaires, **caractérisée en ce que** le médicament est sous la forme de microparticules selon l'une quelconque des revendications 1 à 10, ou une composition selon la revendication 11.